# EUROPEAN PATENT APPLICATION

(11) **EP 3 456 425 A1**
(43) Date of publication of application: **20.03.2019**
(21) Application number: 18194410.9
(22) Date of filing: 14.09.2018
(51) Int. Cl.: B09C 1/10, C02F 3/34, A62D 3/02, C12P 1/02

(54) **DEGRADATION OF POLYCYCLIC AROMATIC COMPOUNDS USING WHITE ROT FUNGHI**

(30) Priority: 14.09.2017 NL 2019549
(71) Applicant: Koninklijke BAM Groep N.V., 3981 AZ Bunnik (NL)
(72) Inventor: VAN BOSTELEN, Martinus, 2396 WP KOUDEKERK AAN DEN RIJN (NL); VAN DRIEL, Roland Antoine Alexander, 7827 TB EMMEN (NL); OTTE, Adrianus Johannes, 7203 HR ZUTPHEN (NL)
(74) Representative: EP&C

(57) **Abstract**

The invention relates to a proces of of degrading polycyclic hydrocarbons from a material containing polycyclic aromatic compounds using white rot fungi, which method comprises the steps of contacting the material containing polycyclic aromatic compounds with the fungi and/or enzymes produced by the fungi, wherein the fungi are selected from the group consisting of *Omphalotus Illudens, Pleurotys Eryngii, Pleurotus Ostreatus, Ganoderma Lucidem* and combinations thereof.

## Description

In general the present invention relates to degradation of polycyclic aromatic compounds (hereinafter also abbreviated to PAC) in a material comprising these compounds using white rot fungi. In particular, the present invention relates to a method of degrading polycyclic aromatic compounds in a waste stream, more particularly reclaimed asphalt granulate that comprises tar.

In the past asphalt containing tar has been used on a wide scale in the construction of roads. It is known that tar comprises polycyclic aromatic hydrocarbons, which are considered carcinogens. In view thereof (inter)national legislation concerning the use of tar in road construction is becoming more stringent. E.g. in the Netherlands tar is not allowed in road pavement since 1990. Until 2001 the use of asphalt granulate comprising tar (TAG) as a recycle product in bottom layer(s) of roads was allowed. Nowadays, complete elimination of asphalt comprising tar from the asphalt chain is a major objective. However, upon renewing older asphalt roads a huge amount of TAG becomes available. This TAG may be reused if it meets certain safety standards. The maximum PAC levels may vary from country to country. E.g. under present legislation in the Netherlands TAG may be reused, if the PAC content is less than 75 mg/kg.

One way to reduce PAC content is by incineration, which is an expensive and environmental unfriendly process. In the Netherlands incineration of TAG is the only way to dispose of TAG that is legally allowed and exploitation is limited to a small group of certified companies.

The use of white rot funghi for biodegradation of polycyclic aromatic compounds found in tar mixtures, in particular *Phanerochaete Chrysosporium*, is described in WO/8801255. This document discloses experiments, wherein degradation of 14C-labeled coal tar constituents, represented by p-cresol, phenanthrene, benzo(a)pyrene and 2-methylnaphthalene, is performed separately in cultures of *Phanerochaete Chrysosporium*.

EP 1537919 A2 discloses a method of microbiological decontamination of demolished road material that contains hazardous compounds like polycyclic aromatic hydrocarbons and phenol compounds. According to this patent application a mixture of at least 5 specifically selected micro-organisms is required, that is to say DSM 8219 (*Pseudomonas Perfetomarina*, *Stutzeri*); DSM 20109 (*Cellulommionas Flavigena*); DSM 291 (*Pseudomonas Putida*); DSM 6506 (*Pseudomonas Fluorescens*); DSM 6612 (*Arthrobacter Oxydans*); and DSM 4762 (*Acidovorac Delafieldii*).

Despite the fact that these approaches as alternatives to incineration are known for a long time, as far as known at present no commercial industrial processes of degrading PAC from TAG has been developed.

Therefore there is a need for (alternative) biodegradation processes that enable the decontamination of polluted material that contains polycyclic aromatic compounds using biodegration.

Accordingly the present invention provides a method of degrading polycyclic hydrocarbons from a material containing polycyclic aromatic compounds using white rot fungi, which method comprises contacting the material containing polycyclic aromatic compounds with enzymes produced by the fungi, the method comprising the steps of
a) culturing the fungi on a substrate in an aqueous medium, wherein enzymes produced by the fungi are incorporated in the aqueous medium;
b) separating the aqueous medium comprising the enzymes produced by the fungi from the substrate;
c) contacting the aqueous medium comprising the enzymes with the PAC containing material.

Separating the aqueous medium comprising the enzymes produced by the fungi from the substrate may also result in separating the aqueous medium comprising the enzymes produced by the fungi from the fungi as well. This is for example the case if the aqueous medium comprising the enzymes is separated from the substrate by filtering and draining the culture solution (the culture solution comprising the aqueous medium, the enzymes and the substrate). The substrate and the fungi then remain on the filter, and the aqueous medium only comprises the enzymes.

JP 2006 314858 A, DE 4104624 C1, EP 1226882 A1, WO 2004/067730 A1, CN 103539493 A, and Kadri et al., J. Env. Sci. 2017, 51, 52-74 all disclose the use of white rot fungi for biodegradation of PAC from contaminated soil. However, none of these documents discloses a method comprising steps a) - c).

For example, JP 2006 314858 A discloses the use of processed products obtained by treating white rot fungi, such as concentrates and dried products obtained from a culture solution for purifying contaminated soil. For example, the concentrate of culture solution may be used. The culture solution comprises the fungi, the substrate and the aqueous medium. Concentrating the culture solution does not include separating the aqueous medium comprising the enzymes produced by the fungi from the substrate. Therefore, JP 2006 314858 A does not disclose step b).

Kadri et al. disclose the use of laccases derived from white rot fungi. However, it is not disclosed in which form the enzymes were used. A method in which the aqueous medium comprising the enzymes produced by the fungi is separated from the substrate, and wherein this same aqueous medium comprising the enzymes is contacted with PAC containing material is therefore not disclosed.

EP 1226882 A1 discloses preparation of a liquid of laccase enzyme from a culture solution by treating the solution with a centrifuge and obtaining the supernatant liquid comprising the laccase. The supernatant was added to aqueous solutions of harmful substances extracted from PAC containing material. Thus, the supernatant was not added directly to the PAC containing material. Direct purification of contaminated soil - thus without first extracting an aqueous solution of the harmful substances from the soil - was also disclosed. However, in these cases, the aqueous medium comprising the enzymes produced by the fungi was not separated from the substrate, but rather the entire culture solution was added to the soil.

Thus, for direct purification of contaminated soil, the prior art teaches that the entire culture solution, thus a solution comprising the enzymes, the fungi as well as the substrate, should be added to the contaminated soil.

However, by performing the process steps a) - c), it is possible to use optimized process conditions for each step, in particular for steps a) and c). Such process conditions comprise e.g. the substrate, which can e.g. be straw, sawdust, wood fibres, etc. as well as temperature, oxygen content and the like. Additives which enhance culturing and colonizing can be added to step a). Examples thereof include carbohydrates, co-enzymes, co-factors including metals such as zinc, copper and iron, gases like oxygen (purging air) and carbon dioxide, and pH-regulating agents.

In an embodiment in step a) the substrate itself comprises a relatively small amount of the PAC containing material to be treated, while in step c) a main volume of the PAC containing material is treated with the aqueous enzyme medium. It has appeared that in this way PAC degradation is more efficient.

In a further advantageous embodiment the aqueous medium used in step c) is recycled to step a) in order to maintain the average amount of enzymes at an effective level. Recycling is also effective in the production of the enzymes. The above selection and colonizing steps can be applied to this further preferred embodiment.

Typically the method is performed batchwise.

A further advantage of splitting the method in separated steps is that the aqueous medium can be produced in one or more centralized production facilities, and be transported to the locations where the PAC containing material is to be treated. For example, a treating facility can be temporarily built in the neighbourhood of a road construction site, where TAG becomes available. An example of such a treating site is a basin, wherein the reclaimed TAG, optionally after size reduction, is temporarily stored and decontaminated. Thereby a reduction of transportation can be realized, because the volume and weight of TAG to be treated is considerably higher than the amount of aqueous medium.

The method according to the invention allows reduction of the PAC level of TAG under natural circumstances as they typically appear in Western Europe, in particular the Netherlands. That is to say within a broad temperature, e.g. 5-37 °C and humidity range without the need of precautionary or additional measures.

In order to allow access to the PAC by the fungi and/or the enzymes produced advantageously the contact area of the material to be treated is large, in other words preferably the material to be decontaminated is a particulate material. E.g. when treating reclaimed asphalt it is advantageous to reduce the size of large lumps (several tens of centimetres) into smaller ones (several centimetres or less, such as less than 10 cm), e.g. by milling and screening.

The fungi can be cultured on a suitable (cellulosic/lignin) substrate, like straw, wood fibres, sawdust and the like. These substrate materials may degrade in water over time, and therefore may clog pipes and nozzles. Therefore, preferably, in step a) the substrate is combined with kapok. Kapok is a natural product and is the fiber from the seed pods of the kapok tree. The kapok fiber principally is an ultra small tube which has a wax coating that is anti-bacterial and anti-fungal. Therefore, the fiber has an increased stability in a wet environment. The resistance of kapok against biological degradation improves the lifespan of the combined substrate as well.

Furthermore, kapok can bind and/or absorb oil and oil products. Especially when the aqueous medium in step a) is contaminated with hydrocarbons, for example because the aqueous medium was recycled from step c), addition of kapok is advantageous, as the kapok removes carbohydrates or residues thereof that are present as contaminants in the aqueous medium. Due to the constant presence of enzymes, these carbohydrates will moreover be degraded.

Preferably, the substrate is combined with kapok in a ratio of 20 : 80 - 60 : 40 kapok vs. substrate, preferably of 30 : 70 - 50 : 50 kapok vs. substrate. Combinations of kapok with substrate were about 25% to 40% more effective in removing hydrocarbons from the aqueous medium than substrates without kapok, which is a result of the absorption of a large volume of hydrocarbons by the kapok.

Preferably, in step a) the substrate is combined with rock wool. Rock wool is a well-known material that is obtained by melting basalt and forming threads which are "spun" into a mat. Although rock wool is not a feeding material for the fungi, the inventors have found that fungi can be introduced on rock wool if further nutrients, i.e. the substrate, for the fungi are provided. Rockwool does not degrade, and the structure of rock wool creates a large surface area. This surface area offers a large colonization space for the fungi and additionally a large contact area for the aqueous medium in order for the aqueous medium to be loaded with enzymes. Rock wool will not degrade under the influence of the aqueous medium, and therefore has a very large life span.

Preferably, the fungi are colonized on a sample of the particulate TAG that is to be treated according to the invention. The particulate TAG may be combined with kapok and/or rock wool as disclosed above. It has appeared that colonizing on the same material results in a strong degradation activity, in particular if the main stream of the contaminated material is contacted by an aqueous treating medium that comprises the enzymes that are produced by the fungi.

More preferably, the fungi are cultured on a substrate which contains the TAG, the most viable parts of the fungus mycelium are selected and those parts are allowed to colonize a new substrate. These selection and colonizing steps are repeated to the point where the fungus (mycelium) is capable of living with the TAG as an only source of nutrient.

In a further preferred embodiment of the method according to the invention the method comprises the steps of contacting the material containing polycyclic aromatic compounds with the fungi and/or enzymes produced by the fungi, wherein the fungi are selected from the group consisting of *Omphalotus Illudens*, *Pleurotys eryngii*, *Pleurotus ostreatus*, *Ganoderma Lucidem* and combinations thereof.

Cultures of these strains have been deposited under the Regulations of the Budapest Treaty in the restricted CBS collection of Westerdijk Fungal Biodiverstiy Institute and have received the following registration nos.:

| | |
|---|---|
| *Pleurotus ostreatus* | CBS 143188 |
| *Pleurotys eryngii* | CBS 143189 |
| *Ganoderma Lucidem* | CBS 143190 |
| *Omphalotus Illudens* | CBS 143191 |

The present inventors have discovered that the abovementioned group of fungi are effective in reducing the PAC level in reclaimed asphalt that contains these compounds. From this group *Pleurotys eryngii*, *Pleurotus ostreatus*, *Ganoderma Lucidem* and combinations thereof are the preferred ones, in particular *Pleurotus ostreatus.*

The method according to the invention can be carried out for removal of PAC from any material that is contaminated or suspected to be contaminated with PAC. Preferably the method according to the invention is applied to asphalt comprising PAC. It is noted that JP 2006 314858 A discloses asphaltene, which is a polycyclic aromatic hydrocarbon contaminant, but the application does not relate to asphalt, which - though the name may be related - is a different product.

Treating a waste stream comprising PAC in order to allow reuse thereof of safe disposal, in particular particulate reclaimed TAG is another preferred application. The asphalt comprising PAC may be derived from a road, such as a road top layer, a wear layer, a bottom layer or a binding layer.

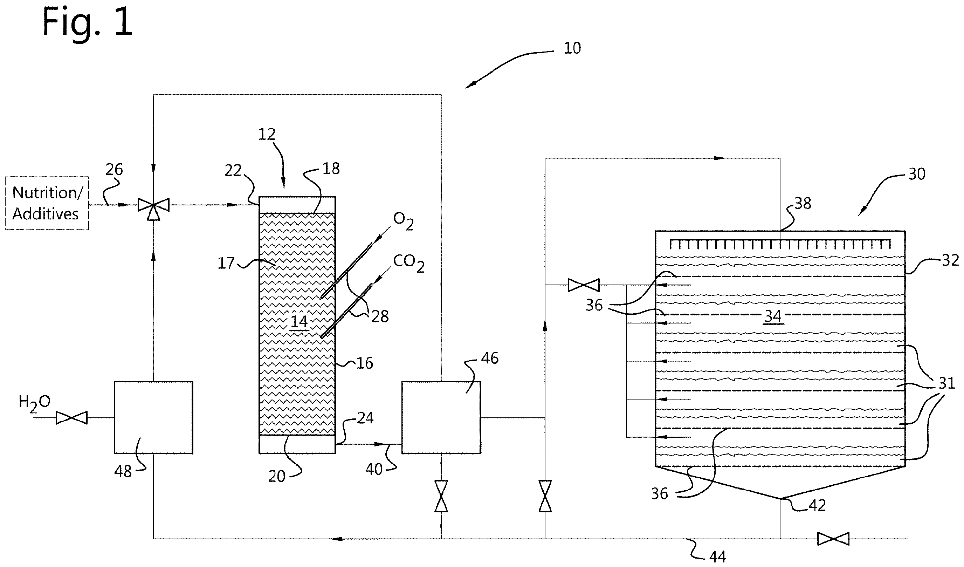

The method according to the invention is further illustrated by means of the drawing, wherein Fig. 1 shows a diagrammatical view of a system for carrying out a preferred embodiment of the invention.

A system 10 for treating contaminated material with enzymes produced by fungi in a liquid treatment medium comprises at least one culturing reactor 12 for culturing and/or colonizing fungi on contaminated material, preferably a sample of the contaminated material to be treated. The reactor 12 comprises a reactor chamber 14 within a housing 16. The chamber 14 is configured to contain an amount of the contaminated material, e.g. the chamber 14 is a column wherein a bed of contaminated material 17 is loosely packed between permeable top and bottom plates 18 and 20. The fungi are deposited on the contaminated material, typically in the form of a suitable substrate, like straw that is colonized with the respective fungi. The fungi and substrate can also be mixed in the contaminated material prior to packing the reaction chamber. Alternating layers of contaminated material and substrate laden with fungi is another way of packing the reaction chamber. Typically the packed contaminated material and substrate with the fungi is periodically sprayed with water and/or the aqueous medium, or permanently or temporarily submerged therein. The housing 16 is provided with a feed 22 for supplying aqueous treatment liquid and a bottom discharge 24 for removing aqueous treatment liquid that is enriched with enzymes produced by the fungi. Line 26 allows to add additives to the culturing reactor 12. Gas supply means 28 for introducing gas in the reactor chamber are provided in the system 10. E.g. lances having a plurality of outflow openings are inserted in the reactor chamber 14 for supplying gas like oxygen or oxygen containing gas and/or carbon dioxide.

Furthermore in the embodiment shown, the system 10 comprises a treatment reactor 30 which is adapted to contain a volume of particulate contaminated material 31. For example, in the embodiment shown the reactor 30 comprises a housing 32 that defines a treatment chamber 34. This chamber 34 is provided with a stack of liquid permeable trays 36 that each hold layered portions of the contaminated material. The chamber 34 is accessible for charging the material to be treated via a door (not shown). The discharge 24 of the culturing reactor 12 is connected to a supply 38 via connecting line 40, through which aqueous medium enriched with the enzymes produced by the fungi in reactor 10 is fed to the treatment reactor 30. A bottom discharge 42 of the treatment reactor 30 connects to the feed 22 of the culturing reactor 12 via return line 44. In the embodiment shown buffer tanks 46 and 48 are incorporated in the connecting line 40 and return line 44. The return line 44 is also connected to buffer tank 46 via line 50. The buffer tank 46 is also connected to the feed 22 of the culturing reactor 12. Arrows indicate the main direction of flow in the system. Other parts like bypasses, filters, pumps, controllers and the like are not shown in Fig. 1.

During operation reactor chamber 14 is charged with an amount of material comprising PAC, a suitable growing substrate and the selected fungi or combination thereof. Water is also added. Reactor chamber 30 is also filled with the material comprising PAC on the trays 36. The aqueous medium comprising the enzymes produced by the fungi in chamber 14 is withdrawn from the reactor chamber 14 via outlet 24 and fed to the treatment reactor 30, where the material comprising PAC is treated by the enzymes. Spent medium is recycled back to the culturing reactor 12. The cycling of treating medium through the system can be on a continuous basis or batchwise until the desired reduction level of the PAC content has been achieved.

The following examples demonstrate the action of the selected fungi.

### Experiments 1

Reclaimed TAG (TAG1/TAG2) was filled into basins in the open air. The PAC compositions (top 10 compounds) of TAG1 and TAG2 are presented in below Table 1.

On the tag a growing substrate comprising a mixture of straw, wood fibres and sawdust that had been colonized with the respective fungi, was positioned. During the experiments periodically the substrate was watered to avoid dehydration. The total experiment time was 150 days. The various fungi tested in experiments 1 were
JOL = Omphalotus illudens
KO = Pleurotus eryngii
PO = Pleurotus ostreatus

Table 1 summarizes also the results in terms of remaining PAC content and reduction. The second and fourth column provide the composition of samples of the TAG1 and TAG2.

**Table 1. Data and results experiment 1**

| Contaminated material | TAG1 | | | | TAG2 | | | | |
|---|---|---|---|---|---|---|---|---|---|
| Fungus | | JOL | KO | PO | | PO | PO | PO | |
| Temperature (avg) [°C] | | 20 | 20 | 20 | | 16 | 16 | 16 | |
| | | | | | | | | | |
| PAC [mg/kg] | | | | | | | | | |
| | | | | | | | | | |
| Naphthalene | 19 | 5.8 | 4.5 | 11 | 29 | 4 | 6.8 | 7 | |
| Phenanthrene | 200 | 72 | 5.7 | 150 | 180 | 53 | 84 | 73 | |
| Anthracene | 29 | 12 | 10 | 23 | 27 | 8.6 | 13 | 12 | |
| Fluoranthene | 180 | 92 | 83 | 160 | 120 | 48 | 80 | 69 | |
| Benzo(a)anthracene | 33 | 19 | 17 | 31 | 20 | 8.5 | 16 | 12 | |
| Chrysene | 30 | 18 | 16 | 29 | 18 | 8.3 | 14 | 12 | |
| Benzo(k)fluoranthene | 7.8 | 4.6 | 5.2 | 8.4 | 3.7 | <2.5 | 4.3 | <2.5 | |
| Benzo(a)pyrene | 16 | 8.9 | 8.7 | 15 | 9.1 | 4 | 6.8 | 6.5 | |
| Benzo(ghi)perylene | 8.4 | 6.3 | 5.2 | 8.4 | 4.8 | <2.5 | 4 | 3.9 | |
| Indenol (1,2,3-cd)pyrene | 7.6 | 4.5 | 5.2 | 8.4 | 4.8 | <2.5 | 3.9 | 3.5 | |
| | | | | | | | | | |
| Sum PAC (10) | 530 | 240 | 210 | 440 | 420 | 140 | 230 | 200 | |
| Reduction (%) | | 55 | 60 | 17 | | 67 | 45 | 52 | |

### Experiments 2

Reclaimed asphalt (TAG3) was loaded into a basin having impermeable side walls and bottom. The top of the basin was open to the air. On top of the reclaimed TAG a layer of straw colonized with *Pleurotus Ostreatus* was provided.

During this experiment a lot of rain water was collected in this basin, which was drained using a pump. Despite this effort anaerobe conditions appeared at certain positions in the basin resulting in mortality of the fungi. Thereafter the fungi were able to recover from these bad conditions.

With the same TAG additional tests were performed. In one test TAG was mixed with mycelium and charged into an open container, and in another one the mycelium was put on top of the TAG in an open container. The fungi used was *Pleurotus Ostreatus.* The results are summarized in Table 2.

**Table 2**

| Contaminated material | TAG3 | 1 Basin | 3 Container Mixed TAG | 4 Container Layered |
|---|---|---|---|---|
| Fungus | | PO | PO | PO |
| Temperature (avg) [°C] | | 20 | 16 | 16 |
| | | | | |
| PAC [mg/kg] | | | | |
| Naphthalene | 47 | 2.6 | <2.5 | <2.5 |
| Phenanthrene | 260 | 13 | 12 | 10 |
| Anthracene | 27 | < 2.5 | <2.5 | <2.5 |
| Fluoranthene | 310 | 20 | 26 | 27 |
| Benzo(a)anthracene | 68 | 4.8 | 5.8 | 5.4 |
| Chrysene | 63 | 4.8 | 6 | 6.2 |
| Benzo(k)fluoranthene | 19 | <2.5 | <2.5 | <2.5 |
| Benzo(a)pyrene | 32 | 3 | 2.6 | 2.9 |
| Benzo(ghi)perylene | 15 | <2.5 | <2.5 | <2.5 |
| Indenol (1,2,3-cd)pyrene | 15 | <2.5 | <2.5 | <2.5 |
| | | | | |
| Sum PAC (10) | 860 | 55 | 61 | 60 |
| Reduction (%) | | 94 | 93 | 93 |

Additional experiments with *Pleurotus Ostreatus* were performed in separated reactors. In a first reactor water was pumped over mycelium and then collected and fed to another reactor into which the TAG was charged. These experiments lasted 4 weeks, during which the TAG was in contact with only the water containing the enzymes. During this period 5 times the aqueous treatment medium was flushed over the TAG for 10 minutes. A reduction of 55% was achieved..

**Table 3.**

| | TAG1 | 5 Mycoreactor Water circulation | 6 Mycoreactor Enzym water |
|---|---|---|---|
| Contaminated material | | TAG1 | TAG 1 |
| Fungus | | PO | PO |
| Time [days] | | 28 | 28 |
| Temperature [°C] | | 16 | 16 |
| | | | |
| Naphthalene | 19 | 4.1 | |
| Phenanthrene | 200 | 87 | |
| Anthracene | 29 | 15 | |
| Fluoranthene | 180 | 86 | |
| Benzo(a)anthracene | 33 | 15 | |
| Chrysene | 30 | 15 | |
| Benzo(k)fluoranthene | 7.8 | 3.9 | |
| Benzo(a)pyrene | 16 | 7 | |
| Benzo(ghi)perylene | 8.4 | 4.4 | |
| Indenol (1,2,3-cd)pyrene | 7.6 | 3.8 | |
| | | | |
| Sum PAC (10) | 530 | 240 | 0.12 |
| Reduction (%) | | 55 | 100 |

### Experiments 3

The straw in above basin 1 of experiments 2 was removed and fresh straw grafted with fungi (*pleurotus ostreatus*) was positioned on top of the TAG3 . The basin was covered with plastic sheet to keep out rainwater. A similar basin (Basin 2) was installed in a shed. TAG (TAG3) was mixed with substrate and mycelium of *Pleurotus Ostreatus.* This basin 2 was equipped with drain pipes which were connected to a collection tube provided with a pump. The drained water could be recycled over the TAG.

Similar experiments were performed in mycoreactors using *Pleurotus Ostreatus.*

The below table 4 summarizes the results.

**Table 4.**

| | Basin 1 | Basin 2 | Myco 1 | Myco 2 | Myco3 | | | |
|---|---|---|---|---|---|---|---|---|
| Contaminated material | TAG 3 | TAG 3 | TAG 3 | TAG 3 | TAG 3 | | | |
| Fungi | PO | PO | PO | PO | PO | | | |
| Time | 190 | 101 | 35 | 35 | 35 | | | |
| Temp [°] | 5 | 6 | 8 | 8 | 8 | | | |
| Reduction [%] | 84 | 86 | 86 | 95 | 92 | | | |

### Experiments 4

In order to evaluate whether an aqueous treating medium comprising enzymes could penetrate into thick layers of TAG various column type reactors were built having different effective heights for packing the TAG and tested

Below table 5 summarizes the results for a column at different heights, wherein TAG (TAG 4 having a total (sum 10) PAC of 1800 mg/kg) is treated with *Pleurotis Ostreatus.* Every two days the medium was drained from the column and passed over mycelium of the fungi. During the test period 3 times the column was subjected to recycling during 4 hours.

**Table 5**

| Depth (cm) | Fungus | Time [days] | Temperature [°C] | Reduction [%] | | |
|---|---|---|---|---|---|---|
| 0 | PO | 28 | 10 | 88 | | |
| 60 | PO | 28 | 10 | 91 | | |
| 120 | PO | 28 | 10 | 92 | | |
| 180 | PO | 28 | 10 | 94 | | |

The lower portions of the column (from about 60 cm and higher) were almost permanently in contact with the treatment medium. This fact may have caused the somewhat higher reduction values at 120 and 180 cm compared to the top of the column at 0 cm.

### Experiment 5

In a mycoreactor TAG (TAG 5 having a total PAC content of 340 mg/kg) was treated with *Ganoderma Lucidem* during 23 days at an average temperature of 17 days. The PAC reduction was 62%.

### Experiment 6

4 Mycoreactors were manufactured, each reactor comprising a housing, an upper spray section having a plurality of nozzles for spraying enzyme water over a lower substrate layer colonized with mycelium of *Pleurotis Ostreatus*. The substrate layer was positioned on a drain plate. The enzyme water was collected in a separate collection tank. Enzyme water was circulated to the upper spray section. From this collection tank enzyme water was also fed to two treatment tanks each containing TAG. In a first treatment tank the enzyme water was fed to the bottom and forced upwards through the TAG and discharged via a weir compartment back to the collection tank. In a second treatment tank the enzyme water was pumped on top of the TAG. In this way the enzyme water supplied percolated through the TAG and then discharged into the collection tank. The whole experiment installation was in the open air and constantly exposed to the prevailing ambient conditions. Due to repeating freezing conditions difficulties were encountered in maintaining the installation in operation and keeping the enzyme water in circulating flow over the fungi mycelia and over the TAG. Despite the non-optimal conditions, in particular the low temperatures, for culturing fungi a significant reduction from 850 mg sum PAC/kg TAG to about 25 mg/kg was achieved in about 13 days of operation.

During this experiment separately small perforated buckets loaded with TAG were also directly placed in the collection tank. In one embodiment a reduction from 850 mg sum PAC/kg TAG to about 280 mg/kg was measured after two days. In another embodiment a reduction from about 330 mg sum PAC/kg TAG to 36 mg sum PAC/kg TAG was determined within 3 days.

## Claims

1. Method of degrading polycyclic hydrocarbons from a material containing polycyclic aromatic compounds using white rot fungi, which method comprises contacting the material containing polycyclic aromatic compounds with enzymes produced by the fungi, the method comprising the steps of
a) culturing the fungi on a substrate in an aqueous medium, wherein enzymes produced by the fungi are incorporated in the aqueous medium;
b) separating the aqueous medium comprising the enzymes produced by the fungi from the substrate;
c) contacting the aqueous medium comprising the enzymes with the PAC containing material.

2. Method according to claim 1, wherein the fungi are selected from the group consisting of *Omphalotus Illudens*, *Pleurotys Eryngii*, *Pleurotus ostreatus*, *Ganoderma Lucidem* and combinations thereof.

3. Method according to claim 2, wherein the fungi are selected from *Pleurotys Eryngii*, *Pleurotus ostreatus*, *Ganoderma Lucidem* and combinations thereof.

4. Method according to any one of the preceding claims, wherein in step a) the substrate is combined with kapok.

5. Method according to claim 4, wherein the substrate is combined with kapok in a ratio of 20 : 80 - 60 : 40 kapok vs. substrate, preferably of 30 : 70 - 50 : 50 kapok vs. substrate.

6. Method according to any one of the preceding claims, wherein in step a) the substrate is combined with rock wool.

7. Method according to any one of the preceding claims, wherein in step a) the fungi are cultured on a substrate comprising the PAC containing material, preferably wherein the fungi are colonized on particulate PAC-containing material..

8. Method according to any one of the preceding claims, wherein after step c) the aqueous medium is at least partially returned to step a).

9. Method according to any one of the preceding claims, wherein the PAC containing material is a waste stream.

10. Method according to any one of the preceding claims, wherein the PAC containing material comprises a tar-containing asphalt, in particular a recovered particulate tar-containing asphalt material from a road surface.
